# EUROPEAN PATENT SPECIFICATION

(11) **EP 1 919 450 B1**
(45) Date of publication and mention of the grant of the patent: **11.06.2014**
(21) Application number: 06779244.0
(22) Date of filing: 31.08.2006
(51) Int. Cl.: A61K 9/127, A61K 45/06, A61K 9/00

(54) **ANTIHISTAMINE- AND CORTICOSTEROID-CONTAINING LIPOSOME COMPOSITION AND ITS USE FOR THE MANUFACTURE OF A MEDICAMENT FOR TREATING RHINITIS AND RELATED DISORDERS**
ANTIHISTAMIN- UND KORTIKOSTEROID-HALTIGE LIPOSOM-ZUSAMMENSETZUNGEN UND IHRE VERWENDUNG ZUR HERSTELLUNG EINES MEDIKAMENTS ZUR BEHANDLUNG VON RHINITIS UND VERWANDTEN ERKRANKUNGEN
COMPOSITION DE LIPOSOME CONTENANT UN ANTIHISTAMINIQUE ET UN CORTICOSTEROIDE ET SON UTILISATION POUR LA FABRICATION D'UN MEDICAMENT POUR TRAITER LA RHINITE ET LES TROUBLES ASSOCIES

(30) Priority: 01.09.2005 US 712822 P
(43) Date of publication of application: 14.05.2008
(73) Proprietor: Meda AB, 170 09 Solna (SE)
(72) Inventor: PERESWETOFF-MORATH, Lena, S-171 65 Solna (SE); CARLSSON, Anders, S-112 38 Stockholm (SE); BJERKE, Torbjørn, S-171 65 Solna (SE)
(74) Representative: Endler, Gabriele
(86) International application number: PCT/GB2006/003222
(87) International publication number: WO 2007/026151

(56) References cited:
- WO-A-03/097100
- WO-A-2005/039533
- US-A1- 2005 112 199

## Description

### Field of the Invention

This invention relates to compositions for use in methods of treating certain inflammatory disorders, such as rhinitis, asthma and chronic obstructive pulmonary disease (COPD), and to processes for the preparation of such compositions.

### Background and Prior Art

There are many diseases/disorders that are inflammatory in their nature. Inflammatory diseases that affect the population include asthma, rhinitis, COPD, inflammatory bowel disease, rheumatoid arthritis, osteoarthritis, conjunctivitis and dermatitis.

Asthma is a disease of the airways that contains elements of both inflammation and bronchoconstriction. Treatment regimens for asthma are based on the severity of the condition. Mild cases are either untreated or are only treated with inhaled ß-agonists which affect the bronchoconstriction element, whereas patients with more severe asthma are typically treated regularly with inhaled corticosteroids which to a large extent are antiinflammatory in their nature.

Allergic and non-allergic rhinitis are common disorders affecting about 30% of the population. Rhinitis has a considerable impact on quality of life. In fact, rhinitis is generally considered to affect the quality of life more so than, e.g., asthma.

Hay fever and perennial allergic rhinitis are characterised by sneezing, rhinorrhea, nasal congestion, pruritus, conjunctivitis and pharyngitis. In perennial rhinitis, chronic nasal obstruction is often prominent and may extend to eustachian tube obstruction.

Oral or local antihistamines are first line treatments, and nasal steroids second line treatments for rhinitis. For most patients, topical corticosteroids and long acting antihistamine agents provide significant relief of symptoms. Antihistamines may also affect non-immunologically (non-IgE) mediated hypersensitivity reactions such as non-allergic rhinitis, exercise induced asthma, cold urticaria, and nonspecific bronchial hyperreactivity.

Cetirizine, [2-{4-[(4-chlorophenyl)phenylmethyl]-1-piperazinyl}ethoxy]acetic acid, is an orally and locally active, potent, long acting peripheral histamine H₁ receptor antagonist. Cetirizine (in the form of the dihydrochloride salt) is one of the most widely used second generation antihistamines for the treatment of rhino-conjunctivitis and urticaria. It is effective, well tolerated and safe when used orally in a dose of 10 mg daily. Sedation and dry mouth do however occur as side effects in orally treated patients. Cetirizine is also approved in children for the treatment of rhinitis.

The main clinical effects of antihistamines include reduced sneezing and rhinorrhea. However, nasal blockage appears to be less responsive. Local administration of antihistamines (such as azelastine and levocabastine) has advantages, including rapid onset of action and fewer side effects.

Local administration of antihistamines (such as azelastine and levocabastine) has advantages, including rapid onset of action and fewer side effects. At present, however, cetirizine dihydrochloride is not an approved medicine for local administration, although it has been administered in that manner in clinical trials.

In one trial (Francillon C, Pécoud A. Effect of nasal spray of cetirizine in a nasal provocation test with allergen. J Allergy Clin. Immunol. 1993:91, Suppl. 2:258 (abstract)), cetirizine nasal spray was found to reduce symptoms and increase nasal peak flow after an allergen challenge. Further, in exercise-induced asthma, a good protective effect was seen when cetirizine mist was administered to the lung with a nebulizer (Ghosh SK, De Vos C, McIlroy I, Patel KR. Effect of cetirizine on exercise induced asthma, Thorax 1991 Apr; 46(4), 242-4).

Some effect was seen on symptoms when cetirizine (presumably as the dihydrochloride) was given as a nasal spray in patients with perennial allergic rhinitis. Concentrations of 0.625, 1.25, and 2.5 mg/mL of cetirizine were sprayed three times a day for two weeks (Clement P, Roovers MH, Francillon C, Dodion P. Dose-ranging, placebo-controlled study of cetirizine nasal spray in adults with perennial allergic rhinitis, Allergy 1994 Sep; 49(8), 668-72). The most common side effects were related to nasal events, although no difference in incidence between the placebo and the cetirizine-treated groups was seen. However, the authors of this article speculated therein that local irritation had an adverse effect on treatment efficacy.

Indeed, due to the irritation of the nasal mucosa by cetirizine, it has been found to be necessary to decrease its immediate exposure in nasal administration. In European Patent No. EP 605 203 B1, it has been reported that this can be achieved by providing cetirizine in form of a composition containing cyclodextrin.

Liposomes (also known as lipid vesicles) are colloidal particles that are prepared from polar lipid molecules derived either from natural sources or chemical synthesis. Such spherical, closed structures composed of curved lipid bilayers, are typically used to entrap drugs, which are often cytotoxic, in order to reduce toxicity and/or increase efficacy. Liposome-entrapped drug preparations are often provided in a dry (e.g. freeze-dried) form, which is subsequently reconstituted with an aqueous solution immediately prior to administration. This is done in order to minimise the possibility of leakage of e.g. cytotoxic drug into aqueous solution and thereby reducing the entrapping effect of the liposome.

Liposomes have also been employed to encapsulate various drug compounds for delivery *via* the nasal route, in order to improve bioavailability or as an adjuvant. Drugs that may be mentioned include tetanus toxoid vaccine, insulin, desmopressin and diphenhydramine hydrochloride (see Türker et al, Review Article: Nasal Route and Drug Delivery Systems, Pharm. World Sci., 2004; 26, 137-142 and the references cited therein), as well as ciprofloxacin, CM3 and salbutamol (see Desai et al, A Facile Method of Delivery of Liposomes by Nebulization, J. Control. Release, 2002; 84, 69-78).

Examples of formulations comprising *inter alia* liposome-encapsulated active ingredients are discussed in US 4,427,649, US 4,839,175, US 5,569,464, EP 249 561, WO 00/38681, WO 88/01862, WO 98/58629, WO 98/00111, WO 03/105805, US 5,049,388, US 5,141,674, US 5,498,420, US 5,422,120, WO 87/01586, WO 2005/039533, US 2005/00112199 and US 6,228,393.

Combination therapies comprising co-administration of antihistamines and corticosteroids are described in WO 97/01337, WO 97/46243, WO 98/48839 and WO 03/049770.

Liposome-entrapped cetirizine has been administered topically to evaluate peripheral antihistaminic activity and systemic absorption in a rabbit model *(*Elzainy et al, Cetirizine from Topical Phosphatidylcholine-Hydrogenated Liposomes, The AAPS Journal, 2004; 6, 1-7, see also Drug Development and Industrial Pharmacy, 2005; 31, 281-291).

WO03097100, WO2005039533, US2005112199 disclose liposomes which may comprise antihistamines and/or a corticoid.

The lipophilic behaviour of the cationic (wherein the anion is chloride), zwitterionic, and anionic forms of cetirizine in buffered aqueous phosphatidylcholine liposome systems containing from about 1 to 33.5 mg/mL of phospholipid has also been studied (Plemper van Balen G et al., Lipophilicity behaviour of the zwitterionic antihistamine cetirizine in phosphatidylcholine liposomes/water systems, Pharm. Res. 2001; 18, 694-701). The aim with the study, in which separate solutions of PBS-diluted egg phosphatidylcholine liposomes were poured into separate compartments of dialysis cells, was to gain insight into the mechanism of interaction of the various electrical species of cetirizine and other drugs with liposomal membranes. The zwitterionic form of cetirizine, which dominates in the pH range of from about pH 4 to about pH 7, and even from about pH 3 to about pH 8, was considered by the authors of this article to be prevented from entry into the liposomal membrane by rendering the formation of lipophilic folded conformers of cetirizine more difficult. In this respect, cetirizine was not entrapped in liposomal membranes for delivery of drug to patients.

Homogeneous pharmaceutical compositions containing cetirizine and a polar lipid liposome have been disclosed in international patent application WO 2005/107711.

However, none of the above-mentioned references disclose or suggest any liposomal pharmaceutical composition comprising a combination of corticosteroid and antihistamine.

Surprisingly, we have found that the irritation that may be associated with (e.g. nasal) administration of certain antihistaminic active ingredients, including cetirizine, may be reduced by way of use of homogeneous pharmaceutical compositions comprising such an active ingredient, a polar lipid liposome and a pharmaceutically acceptable carrier.

According to the present invention, there is provided a homogeneous pharmaceutical composition according to claim 1 suitable for the treatment of, for example, rhinitis comprising, as active ingredients, an antihistamine and a corticosteroid, as well as polar lipid liposomes and a pharmaceutically-acceptable aqueous carrier, which compositions are referred to hereinafter as "the compositions of the invention".

The skilled person will appreciate that the relevant active ingredients are employed in compositions of the invention in pharmacologically-effective amounts (*vide infra*). The term "pharmacologically-effective amount" refers to an amount of relevant active ingredient, which is capable of conferring the desired therapeutic effect on a treated patient, whether administered alone or in combination with the other, or another, active ingredient. Such an effect may be objective (i.e. measurable by some test or marker) or subjective (i.e. the subject gives an indication of, or feels, an effect).

By "pharmaceutical compositions" we include compositions that are suitable for use in direct administration to mammals, and especially humans. In this respect, the term is intended to encompass formulations that include only components that are regarded in the art as suitable for administration to mammalian, and especially human, patients. In the context of the present invention, the term may also mean that the compositions of the invention are in a form of a liquid that is ready-to-use, directly from the shelf, and not a formulation in which drugs are encapsulated inside liposomes requiring reconstitution shortly prior to administration in order to avoid leakage of drugs from liposomes into an aqueous carrier.

By "homogeneous" we include not only that the compositions of the invention comprise liposomes dispersed evenly throughout the aqueous carrier, but further that active ingredients are distributed throughout the whole composition. This means that no process steps are performed that may serve to increase entrapment, or encapsulation, efficiency of active ingredient(s) into liposomes, such as remote loading (an 'active' loading method in which preformed liposomes and active ingredient(s) are incubated under a transmembrane gradient, e.g. pH, resulting in high encapsulation efficiency), and/or that, following formation of a mixture comprising liposomes and active ingredients in aqueous medium, active ingredients that are not encapsulated within liposomes are not removed following liposome formation. This may, in the case of certain compositions of the invention, result in a substantially similar concentration of one or more of the active ingredients in the relevant aqueous medium, whether that medium is located inside or outside of the liposomal structures. By "substantially similar", we include that the concentration may vary by about ±50%, such as about ±40%, preferably about ±30%, more preferably about ±20% and particularly about ±10% (when comparing concentrations inside and outside of the liposomal structures) at room temperature and atmospheric pressure. Drug concentration profiles may be measured by standard techniques known to the skilled person, such as ³¹P-NMR. For example, a standard *in situ* probing technique, or a technique that involves separation of the liposomal fraction from the free aqueous carrier and measurement of the amount/concentration of active ingredient(s) associated .with each fraction may be employed. Separation may be accomplished by centrifugation, dialysis, ultrafiltration, or gel filtration.

It is preferred that the compositions of the invention further include a pharmaceutically-acceptable buffer capable of providing a pH of from about pH 4 to about pH 8, preferably from about pH 5 to about pH 7. Appropriate buffers include those that will not interfere with the formation of liposomes, such as a phosphate (e.g. disodium phosphate, dipotassium phosphate, sodium dihydrogen phosphate, potassium dihydrogen phosphate or phosphoric acid plus base), citrate (e.g. sodium citrate or citric acid plus base), or acetate (e.g. sodium acetate or acetic acid plus base) buffer, which is capable of maintaining a pH within the above-specified ranges. Buffers may be employed in an amount that is suitable to provide for the above-mentioned effects and such will be appreciated by the skilled person without recourse to inventive input. Appropriate quantities are for example in the range of about 1 mg/mL to about 30 mg/mL.

Compositions of the invention find particular utility in the treatment of allergic disorders, such as asthma and rhinitis, as well as COPD.

Compositions of the invention find particular utility in the treatment of rhinitis. The term "rhinitis" will be understood to include any irritation and/or inflammation of the nose, whether allergic or non-allergic, including seasonal rhinitis (e.g. caused by outdoor agents such as pollen; hay fever) and/or perennial rhinitis (e.g. caused by house dust mites, indoor mould etc), as well as the symptoms thereof.

Corticosteroids that may be mentioned include alclometasone, beclometasone, betamethasone, budesonide, ciclesonide, clobetasol, clobetasone, deflazacort, deprodone, dexamethasone, diflucortolone, fluocinolone, etiprednol, flunisolide, fluocinonide, fluocortolone, fluprednidene, flurometholone, fluticasone, halcinonide, hydrocortisone, KSR 592, loteprednol, methylprednisolone, mometasone, prednisolone, rimexolone and triamcinolone and commonly employed salts thereof.

More preferred corticosteroids include budesonide, ciclesonide, fluticasone, triamcinolone and mometasone and commonly employed salts thereof, and particularly budesonide and fluticasone (e.g. the latter in the form of a salt, such as a propionate salt).

Antihistamines may comprise H₁ receptor antagonists. H₁ histamine receptor antagonists that may be mentioned include acrivastine, alimemazine, anatazoline, astemizole, azatadine, azelastine, bamipine, bepotastine, bromazine, bromopheniramine, buclizine, carbinoxamine, cetirizine, chlorocyclizine, chloropyramine, chlorophenamine, cinnarizine, clemastine, clemizole, clocinizine, cyclizine, cyproheptadine, deptropine, desloratadine, dexchlorpheniramine, dimenhydrinate, dimetindene, dimetotiazine, diphenhydramine, piphenylpyraline, doxylamine, ebastine, efletirizine, embramine, emedastine, epinastine, fexofenadine, flunarizine, homochlorocyclizine, hydroxyzine, isothipendyl, levocarbastine, levocetirizine, loratadine, mebhydroline, meclozine, mepyramine, mequitazine, methdilazine, mizolastine, niaprazine, olopatadine, oxatomide, oxomemazine, pemirolast, phenindamine, pheniramine, phenyltoloxamine, pimethixene, pipinhydrinate, promethazine, propiomazine, quifenadine, rupatadine, setastine, terfenadine, thenyldiamine, thiethylperazine, thonzylamine, tolpropamine, trimethobenzamine, tripelennamine, triprolidine and tritoqualine and commonly employed salts thereof.

More preferred antihistamines include loratadine and, more particularly, azelastine, fexofenadine, more preferably levocetirizine and, most preferably, cetirizine and commonly employed salts thereof.

Unless above-mentioned active ingredients are already provided in diasteromerically (or enantiomerically) enriched form, individual diastereoisomers and enantiomers of active ingredients, and mixtures of such diastereoisomers/enantiomers may be used in compositions of the invention.

Furthermore, any pharmaceutically-acceptable salt of an active ingredient, as well as the free base form thereof may be used in the manufacture of compositions of the invention. Preferred salts include acetate salts, acetonate salts, aluminium salts, ammonium salts, arginine salts, bromide salts, butyrate salts, calcium salts, chloride salts, choline salts, citrate salts, diethanolamine salts, diethylamine salts, dipropionate salts, embonate salts, ethanolamine salts, ethylenediamine salts, formate salts, fumarate salts, fuorate salts, hydrobromide salts, hydrochloride salts, imidazole salts, lactate salts, lysine salts, magnesium salts, malate salts, maleate salts, malonate salts, meglumine salts, mesilate salts, morpholine salts, nitrate salts, phosphate salts, piperazine salts, potassium salts, propionate salts, sodium salts, succinate salts, sulfate salts, tartrate salts, teoclate salts, *para*-toluenesulfate salts, triethanolamine salts, triethylamine salts, valerate salts, etc and/or as described in "Handbook of Pharmaceutical Salts", Eds. Stahl and Wermuth, Wiley, 2002, Chapter 12.

When the antihistamine active ingredient that is employed is cetirizine, preferred salts include chloride salts, hydrochloride (e.g. dihydrochloride) salts and nitrate (e.g. dinitrate) salts of cetirizine. More preferred salts include cetirizine dinitrate and, especially, cetirizine dihydrochloride.

The absolute and relative amounts of active ingredients that may be employed in preparation of compositions of the invention may be determined by the physician, or the skilled person, in relation to what will be most suitable for an individual patient. This is likely to vary with the nature of the active ingredients that are employed, the severity of the condition that is to be treated, as well as the species, age, weight, sex, renal function, hepatic function and response of the particular patient to be treated. It is preferred however that the compositions of the invention comprise active ingredients (or salts), in a total amount of from about 0.1 mg/mL to about 200 mg/mL calculated on the free-base forms.

The total amounts of the active ingredients that are present may be sufficient to provide a daily dose per unit dosage that is appropriate for the respective active ingredients that are employed. For example, this may be in the range about 20 µg to about 200 mg.

Individual concentrations and dosing regimens for antihistamines are in the ranges of about 0.5 (such as about 0.7, e.g. about 1 mg/mL) to about 150 mg/mL, and about 0.2 mg to about 200 mg, respectively. Individual concentrations and dosing regimens for corticosteroids are in the ranges of about 50 µg to about 1,500 µg/mL, and about 20 (e.g. about 50) µg to about 1,600 µg, respectively.

The skilled person will appreciate that compositions of the invention may be dosed once or more times daily in one or more administrations in order to provide the aforementioned daily dose(s).

When the antihistamine active ingredient that is employed is cetirizine, compositions of the invention comprise cetirizine or a salt thereof in an amount of from about 1 mg/mL to about 30 (e.g. about 25, such as about 23) mg/mL calculated on the zwitterionic form, preferably in an amount of from about 5.5 mg/mL to about 22 mg/mL. A further preferred range is between about 6 mg/mL and about 15 mg/mL, such as about 8 mg/mL to about 12 mg/mL. In such a case, the total amount of cetirizine that may be present may be sufficient to provide a daily dose of cetirizine per unit dosage that is in the range about 4 mg to about 20 mg, such as about 5 mg to about 15 mg, more preferably about 7 mg to about 12 mg and most preferably about 8 mg to about 10 mg.

The above-mentioned dosages of active ingredients are exemplary of the average case; there can, of course, be individual instances where higher or lower dosage ranges are merited, and such are within the scope of this invention.

The term "liposome" will be well understood by those skilled in the art to include a structure consisting of one or more concentric spheres of polar lipid bilayers separated by water or aqueous buffer compartments.

Liposomes may be prepared by various methods using solvents, reduced pressure, two-phase systems, freeze drying, sonication etc. described, for instance, in Liposome Drug Delivery Systems, Betageri G V et al., Technomic Publishing AG, Basel, Switzerland, 1993.

The term "polar lipid" will be well understood by the skilled person to include any lipid with a polar head-group and two fatty acid residues, which is capable of forming liposomes.

Polar lipids, such as those described hereinafter, may be of a natural and/or a synthetic/semi-synthetic origin. Mixtures of natural and synthetic/semi-synthetic polar lipids may also be employed in compositions of the invention.

Polar lipids that may be employed in compositions of the invention may thus be based on, for example, phospholipids, and in particular phosphatidylcholine (PC), phosphatidylglycerol (PG), phosphatidylinositol (PI), phosphatidic acid (PA), phosphatidylserine (PS), or mixtures thereof.

Phospholipids that may be employed in compositions of the invention comprise polar and non-polar groups linked to a backbone entity carrying hydroxyl groups, such as glycerol.

Phospholipids may also be represented by the general formula I, 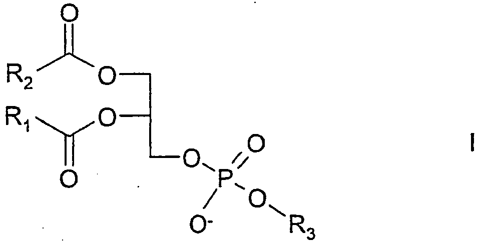 wherein R₁ and R₂ independently represent a saturated or unsaturated (e.g. alkenyl), branched or straight chain alkyl group having between 7 and 23 carbon atoms, preferably between 11 and 19 carbon atoms; and R₃ represents an amide or ester bonding group, such as
-CH₂-CH(OH)-CH₂OH (phosphatidylglycerol),
-CH₂-CH₂-N(CH₃)₃ (phosphatidylcholine),
-CH₂-CH₂- NH₂ (phosphatidylethanolamine),
-H (phosphatidic acid), or
-CH₂-CH(NH₂)-COOH (phosphatidylserine).

The phospholipid may be of natural origin. Natural phospholipids are preferably membrane lipids derived from various sources of both vegetable (e.g. rapeseed, sunflower, etc., or, preferably, soybean) and animal origin (e.g. egg yolk, bovine milk, etc.). Phospholipids from soybean, a major source of vegetable phospholipids, are normally obtained from the by-products (i.e. lecithins) in the refining of crude soybean oil by the degumming process. The lecithins are further processed and purified using other physical unit operations, such as fractionation and/or chromatography. Other phospholipids may be obtained, for example, by pressing various suitable seeds and grains, followed by solvent extraction and then further processing as described above. Phospholipids of natural origin that may be mentioned include for example those that are available under the tradenames Lipoid S75, Lipoid S100 and Lipoid S75-3N (Lipoid GmbH, Germany), which are all blends of several different phospholipids that are found in soybean.

The phospholipid may alternatively be of synthetic or semi-synthetic origin (i.e. prepared by chemical synthesis). For example, a multi-step chemical synthetic approach may be used in order to obtain the key phospholipid intermediates, 1,2-diacylglycerol, from (*S*)-1,2-isopropylideneglycerol, the latter providing the glycerol backbone that is characteristic of phospholipids. 1,2-Diacetylated phospholipids may then be obtained when the corresponding polar head group is attached *via* chemical synthesis to the 1,2-diacylglycerol intermediate. Generally, however, the origin of glycerol and the fatty acids used in the various steps may be of both natural and synthetic origin. Synthetic and/or semi-synthetic phospholipids that may be mentioned include dilaurylphosphatidylcholine (DLPC), dimyristolphosphatidylcholine (DMPC), dipalmitoylphosphatidylcholine (DPPC), dilaurylphosphatidylglycerol (DLPG), dimyristolphosphatidylglycerol (DMPG), dioleoylphosphatidylcholine (DOPC) and dioleoylphosphatidylglycerol (DOPG). DOPC and DMPC are preferred, for example in combination with one or more of the Lipoid phospholipids mentioned hereinbefore.

The polar lipid may alternatively comprise or, more preferably, consist of a glycolipid. In the context of the present invention, the term "glycolipid" designates a compound containing one or more monosaccharide residues bound by a glycosidic linkage to a hydrophobic moiety such as an acylglycerol, a sphingoid or a ceramide (N-acylsphingoid).

A glycolipid may be a glycoglycerolipid. In the context of the present invention, the term "glycoglycerolipid" designates a glycolipid containing one or more glycerol residues. According to a preferred aspect of the invention, the glycoglycerolipid comprises, or consists of, galactoglycerolipid, more preferably a digalactosyldiacylglycerol of the general formula II, 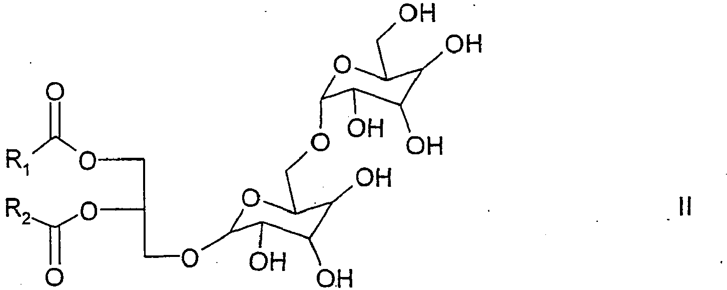 wherein R₁ and R₂ are as hereinbefore defined.

The glycolipid may alternatively be a glycosphingolipid. In the context of the present invention, the term "glycosphingolipid" designates a lipid containing at least one monosaccharide residue and either a sphingoid or a ceramide. The term may thus comprise neutral glycophingolipids, such as mono- and oligoglycosylsphingoids as well as oligo- and, more preferably, monoglycosylceramides. The term additionally comprises acidic glycosphingolipids such as sialoglycosphingolipids, uronoglycosphingolipids, sulfoglycosphingolipids, phosphoglycosphingolipids, and phosphonoglyco-sphingolipids. The glycosphingolipid can be ceramide, monohexosylceramide, dihexosylceramide, sphingomyelin, lysosphingomyelin, sphingosine, or a mixture thereof. Preferably the glycosphingolipid is sphingomyelin or a product derived therefrom. The sphingomyelin content is preferably established by chromatographic methods. Sphingomyelin may be extracted from milk, preferably bovine milk, brain, egg yolk or erythrocytes from animal blood, preferably sheep. For the avoidance of doubt, synthetic and semi-synthetic sphingolipids are comprised by the invention.

The glycolipid may alternatively be a glycophosphatidylinositol. In the context of the present invention, the term "glycophosphatidylinositol" designates a glycolipid containing saccharides glycosidically linked to the inositol moiety of phosphatidylinositols.

Preferred glycolipids include digalactosyldiacylglycerol (DGDG).

It is preferred that the polar lipid is based on a phospholipid and, more particularly, a phospholipid derived from soybean (e.g. Lipoid S100 or Lipoid S75-3N).

Preferred polar lipids (such as phospholipids) are those that swell to a measurable degree in water and/or those which are capable of spontaneous liposome formation.

If the polar (e.g. phospho-) lipid does not swell spontaneously in water, the skilled person will appreciate that it is nevertheless possible to obtain liposomes by adding a more polar, swellable (e.g. phospho-) lipid, such as an anionic (e.g. phospho-) lipid (e.g. phosphatidylglycerol).

Liposome formation may be performed at above about 0°C (e.g. room temperature) if the phase transition temperature of the acyl chains (chain melting; gel-to-liquid crystals) is below the freezing point of water.

Whichever polar lipid substance (or combination thereof) is used, suitable total amounts/concentrations of lipid(s) that may be employed in preparation of a composition of the invention are in the range of about 10 mg/mL to about 120 mg/mL. Compositions of the invention that may be mentioned include those in which, when the polar lipid comprises phospholipid (whether in combination with another lipid or otherwise), the amount of phospholipid(s) in the composition is from about 10 (e.g. about 17, such as about 20) mg/mL to about 120 mg/mL, more preferably from about 25 (e.g. about 35) mg to about 100 (e.g. about 70, such about 50, e.g. about 40) mg/mL. Typical ranges that may be mentioned include from about 25 (e.g. 27) mg/mL to about 50 mg/mL (e.g. 45 or, more particularly, 35 mg/mL). Further, the total amount of phospholipid (when the polar lipid comprises phospholipid) is preferably in the range from about 10 mg to about 80 mg (such as from about 17 (e.g. 20) mg to about 70 (e.g. 40) mg).

Compositions of the invention may also comprise an antioxidant, such as α-tocopherol, ascorbic acid, butylated hydroxyanisole, butylated hydroxytoluene, citric acid, fumaric acid, malic acid, monothioglycerol, propionic acid, propyl gallate, sodium ascorbate, sodium bisulfite, sodium metabisulfite, potassium metabisulfite, sodium sulfite, tartaric acid or vitamin E. Preferred antioxidants include butylated hydroxytoluene, α-tocopherol, ascorbic acid and butylated hydroxyanisole.

According to the invention a chelating agent may be used to reduce the metal ion catalysed oxidation of phospholipid and/or active ingredient(s). Examples of useful chelating agents are ethylenediaminetetraacetic acid (EDTA) and salts thereof (e.g. sodium or potassium EDTA), ethylenediaminetriacetic acid and diethylenetriaminepentaacetic acid (DTPA). It is also possible to use other agents that protect the composition of the invention and, in particular, any unsaturated fatty acid residues that may be present therein, from oxidation. Preferred chelating agents include EDTA and salts thereof.

The composition of the invention can comprise one or more preservatives. Examples of common preservatives for liquid pharmaceutical compositions are benzalkonium chloride, benzoic acid, butylated hydroxyanisole, butylparaben, chlorbutanol, ethylparaben, methylparaben, propylparaben, phenoxyethanol or phenylethyl alcohol. Preferred preservatives include benzalkonium chloride. Other preservatives that may be mentioned include sorbic acid.

In order to retain the composition of the invention at its application site it may also comprise viscosity-increasing agent such as, for instance, hydrophilic polymers like polyethyleneglycol, or crosslinked polyvinylpyrrolidone and/or cellulose derivatives such as hydroxypropylmethyl cellulose. Viscosity increasing agents may also function as protective colloids to physically stabilise the composition of the invention prior to administration. Preferred protective colloids include hydroxypropylmethyl cellulose and, more particularly, polyethylene glycol.

Compositions of the invention may also comprise flavourings (e.g. lemon, menthol or peppermint powder) and/or sweeteners (e.g. neohesperidin).

Compositions of the invention may also comprise tonicity-modifying agents, such as sodium chloride, potassium chloride, glycerol, glucose, dextrose, sucrose, mannitol, etc.

Optional additives, including buffering agents, preservatives, viscosity-increasing agents, antioxidants, tonicity-modifying agents and chelating agents should be selected, in terms of their identity and the amounts employed, keeping in mind that their detrimental effect on liposome stability should be kept at a minimum. For a given agent this can be ascertained by simple experiments, which are well within the understanding of the skilled person. Suitable amounts of such ingredients are however in the range about 0.01 mg/mL to about 10 mg/mL. It is preferred that the compositions of the invention contain at least one preservative, antioxidant, chelating agent, buffering agent and/or viscosity-increasing agent. Suitable amounts of any/all of these optional additives include from about 0.02 to about (e.g. about 3) mg/mL (e.g. from about 0.1 to about 2 mg/mL).

There is also provided a process for preparing compositions of the invention. We have surprisingly found that liposomes may be prepared by direct swelling of the polar lipids in an aqueous medium without the addition of any other excipients such as charged lipids and/or surfactants etc., which are normally required.

According to a further aspect of the invention, there is provided a process for preparing a composition of the invention, which process comprises:
(a) mixing together, in an aqueous medium, a corticosteroid, an antihistamine and a polar lipid, or a mixture of polar lipids, that is/are swellable in aqueous media; and
(b) homogenising the preparation.

Step (a) of the above-mentioned process is preferably carried out in the presence of suitable agitation (e.g. stirring).

The aqueous medium may comprise water, saline or preferably a buffer solution. Polar lipid(s), corticosteroid and antihistamine (and excipients if and when employed) may be added to the aqueous medium in any order during step (a).

Preferably the pH of the preparation is adjusted, for example prior to the homogenisation step (b) above, to a desired value within the range of from about pH 4 to about pH 8, preferably from about pH 5 to about pH 7, by adding an acid or a base (e.g. hydrochloric acid and/or sodum hydroxide at an appropriate concentration (e.g. 1M)).

Water, saline or buffer solution may be added, for example prior to the homogenisation step (b) above and/or after the pH adjusting step mentioned above, to the preparation to obtain a desired final batch volume.

Solutions/liquids may be purged with nitrogen or argon at a suitable stage in the above process, if and as appropriate.

In the context of the present invention, a lipid may be said to be swellable in aqueous media if, when placed in contact with such a medium, it swells to a measurable degree.

The formation of the liposomes of the invention may be facilitated by the spontaneous swelling of the polar lipid in water forming a lamellar liquid crystalline phase having a maximum water content of about 35% by weight or higher depending on the nature of the polar lipid. Depending on the lipid or lipid mixture used and other conditions, spontaneous formation of liposomes may be achieved when excess water is added to this lamellar phase. If spontaneous formation is not achieved, the formation of liposomes may be accomplished by the mechanical dispersion step (i.e. the homogenisation step (b) of the above process) of the lamellar liquid-crystalline phase in excess water.

Homogenisation/dispersion methods include vigorous mechanical mixing or high speed homogenisation, for instance by means of an Ultra Turrax® (Jankel & Kühnke, Germany). Shaking, vortexing and rolling may also be performed as part of the homogenisation step of the above process.

A homogeneous size distribution of the liposomes of the invention may be desirable and may be obtained by extrusion through a membrane filter, such as one made of polycarbonate, with a pore size of about 100 nm. Membrane filters may be procured from Avestin Inc., Canada.

A reduced average liposome size and narrowed liposome size distribution may preferably also be obtained when the liposomal dispersion is subjected to high-pressure homogenisation with a suitable homogeniser (Rannie APV, type 7.30 VH, Rannie AS, Denmark) at, for example, between about 300 bar and about 1000 bar, such as between about 400 bar and about 900 bar, e.g. about 500 to about 800 bar for between about 4 and about 8 (e.g. 7, such as 6) cycles.

We have found that the presence of certain active ingredients (e.g. cetirizine) may result in a reduction of liposome size. Smaller liposomes are generally advantageous because they are more stable physically and, due to their higher surface area/volume ratio, are more easily resorbed by the mucosa.

We prefer that the diameter of liposomes in compositions of the invention is less than about 200 nm (e.g. between about 40 to about 100 nm), as measured by, for example, laser diffraction or dynamic light scattering.

Furthermore, the above-mentioned process for the preparation of compositions of the invention does not normally require conventional treatment with organic solvents such as chloroform or dichloromethane. However, it may be appropriate and/or necessary to treat lipids and/or corticosteroids with organic solvent prior to the addition of, or addition of them to, the aqueous solvent. For example, the lipids and/or corticosteroids may be dissolved in an organic solvent or solvent mixture. The solution may then be deposited on the surfaces of a round-bottomed flask as the solvent is removed by rotary evaporation under reduced pressure. An excess volume of aqueous buffer containing drug(s) may then be added to the dry thin film of lipids, which may then be allowed to swell to form liposomes. In other cases, if any active ingredient is significantly insoluble in water and/or phospholipid, it may be necessary to dissolve it and the phospholipid in an organic solvent prior to addition of the aqueous phase. Again, organic solvent may be removed (e.g. in *vacuo*) prior to addition of the aqueous phase.

The compositions of the invention are useful in the treatment of any indication for which the relevant active ingredient(s) is/are known to be effective, for example those specifically listed for those ingredients in question in Martindale "The Complete Drug Reference", 34th Edition, Royal Pharmaceutical Society (2005).

According to a further aspect of the invention, there is provided a method for the treatment of rhinitis, of asthma and/or of COPD, comprising the administration of a pharmacologically-effective amount of a composition of the invention to a person suffering from or susceptible to that disorder.

For the avoidance of doubt, by "treatment" we include the therapeutic treatment, as well as the symptomatic treatment, the prophylaxis, or the diagnosis, of a condition.

Although compositions of the invention may be administered by any known route, including parenterally, topically and/or perorally, they may normally be administered transmucosally and, more particularly, nasally, ocularly and pulmonarily. For example, compositions of the invention may be administered by way of a nasal spray, nasal drops and/or eye drops. It is also possible to administer compositions of the invention as a fine mist to the lungs by nebulization. For nasal administration, any state-of-the-art device suitable for producing sprays of aqueous liposomal dispersions may be used.

Such formulations may be prepared in accordance with standard and/or accepted pharmaceutical practice.

Wherever the word "about" is employed herein in the context of dimensions (e.g. pH values, sizes, temperatures, pressures, etc.) and amounts (e.g. amounts, weights and/or concentrations of individual constituents in a composition or a component of a composition, proportions of active ingredient(s) inside/outside the liposomal structures, absolute doses of active ingredient(s), etc.), it will be appreciated that such variables are approximate and as such may vary by ± 10%, for example ± 5% and preferably ± 2% (e.g. ± 1%) from the numbers specified herein.

The compositions of the invention, and the above-mentioned process that may be employed for their preparation, have the advantages that are mentioned hereinbefore. In particular, compositions of the invention may reduce the incidence of inconvenient side-effects (and in particular irritation) that are often observed with e.g. nasally-administered formulations.

Compositions of the invention are easy to manufacture and enable the production of liposomal-based formulations that are in a ready-to-use form, avoiding the need for reconstitution prior to administration.

Compositions of the invention may also have the advantage that they may be prepared using established pharmaceutical processing methods and employ materials that are approved for use in foods or pharmaceuticals or of like regulatory status.

Compositions of the invention may also have the advantage that they may be more efficacious than, be less toxic than, be longer acting than, be more potent than, produce fewer side effects than, be more easily absorbed than, and/or have a better pharmacokinetic profile than, and/or have other useful pharmacological, physical, or chemical properties over, pharmaceutical compositions known in the prior art, whether for use in the treatment of inflammatory disorders such as rhinitis, asthma and/or COPD, or otherwise.

The invention is illustrated by way of the following examples.

General procedure. For weights and volumes reference is made to the tables below. A buffer solution is prepared by dissolving the applicable buffer salts in 160 mL water (80% of the total batch volume) in a 200 mL volumetric flask. The weighed amounts of applicable excipients are added and dissolved by stirring with a magnetic stirrer. The weighed amount of the relevant antihistamine is added and dissolved by stirring. Appropriate phospholipid(s), such as Lipoid S100 (and DMPC (if employed)) are separately weighed, mixed and added to the solution. Finally, the weighed amount of the relevant corticosteroid is added and stirring is continued until a well dispersed suspension has formed; the desired pH is adjusted with 1.0 M NaOH and/or 1.0 M HCl. The volume of the preparation is then brought to the final batch volume of 200 mL. The preparation is transferred to a high pressure homogeniser (Rannie APV, type 7.30 VH, Rannie AS, Denmark) and homogenised at 800 bar for 7 cycles. Aliquots of the thus obtained composition are removed from the collecting vessel and transferred to glass vials.

The above procedure was employed in order to prepare final compositions as outlined in Examples 1 to 4 below. Where appropriate, the quantities of the components were scaled up appropriately (e.g. in the case of Examples 1 to 4, multiplied by 200). The procedures for Examples 5 and 6 are described separately below.

### Example 1

| Ingredient | Quantity |
|---|---|
| Cetirizine dihydrochloride | 11.1 mg |
| Budesonide | 320 µg |
| DMPC | 8.05 mg |
| Lipoid S100 | 26.95 mg |
| BHT | 0.02 % |
| Benzalkonium chloride | 0.2 % |
| Citric acid, anhydrous | 19.2 mg |
| Sodium hydroxide, solid (NaOH) | 8.4 mg |
| 1M NaOH and/or 1 M HCl | to pH 5.5 |
| Purified water | to 1 mL |

### Example 2

| Ingredient | Quantity |
|---|---|
| Cetirizine dihydrochloride | 11.1 mg |
| Fluticasone propionate | 125 µg |
| DMPC | 8.05 mg |
| Lipoid S100 | 26.95 mg |
| BHT | 0.02 % |
| Benzalkonium chloride | 0.2 % |
| Citric acid, anhydrous | 19.2 mg |
| Sodium hydroxide, solid (NaOH) | 8.4 mg |
| 1M NaOH and/or 1 M HCl | to pH 5.5 |
| Purified water | to 1 mL |

### Example 3

| Ingredient | Quantity |
|---|---|
| Loratadine | 1.0 mg |
| Budesonide | 320 µg |
| Lipoid S100 | 35.0 mg |
| Benzalkonium chloride | 0.2 mg |
| Citric acid, anhydrous | 19.2 mg |
| Sodium hydroxide, solid (NaOH) | 8.4 mg |
| 1M NaOH and/or 1 M HCl | to pH 5.0 |
| Purified water | to 1 mL |

### Example 4

| Ingredient | Quantity |
|---|---|
| Loratadine | 1.0 mg |
| Fluticasone propionate | 125 µg |
| DMPC | 8.05 mg |
| Lipoid S100 | 26.95 mg |
| Citric acid, anhydrous | 19.2 mg |
| Sodium hydroxide, solid (NaOH) | 8.4 mg |
| 1M NaOH and/or 1 M HCl | to pH 5.0 |
| Purified water | to 1 mL |

The commercially available nasal antihistamine azelastine (registered trademarks including Azelvin®, Azosin®, Astelin®, Lastin® and Rhinolast®) was formulated using the quantities and steps outlined below.

### Example 5

1. 160 mL of azelastine solution for nasal administration (Lastin^{®}) containing 0.9 mg/mL azelastine was transferred to a 200 mL volumetric flask.
2. 7 g of soy bean phospholipid (Lipoid S100, Lipoid GmbH, Germany) was added.
3. 64 mg of budesonide was added and stirring was continued until a well dispersed suspension had formed (overnight).
4. The volume was brought to 200 mL by the addition of more azelastine solution (see step 1 above).
5. The pH was checked.
6. The solution was homogenised for 7 cycles at 800 bar as described in the general procedure above.

### Example 6

The general procedure described in Example 5 above was followed, except that, in place of step (3), 25 mg of fluticasone propionate was added in place of the budesonide.

## Claims

1. A homogeneous pharmaceutical composition comprising an antihistamine, a corticosteroid, a polar lipid liposome and a pharmaceutically-acceptable aqueous carrier, wherein the concentration of active ingredient in the aqueous carrier is substantially similar, whether located inside or outside the liposomal structures and varies by +-10% when comparing concentrations inside and outside of the liposomal structures.

2. A composition as claimed in Claim 1 which further includes a pharmaceutically-acceptable buffer capable of providing a pH of from about pH 4 to about pH 8.

3. A composition as claimed in Claim 2, wherein the pH range is about pH 5 to about pH 7.

4. A composition as claimed in Claim 2 or Claim 3, wherein the buffer is a phosphate, citrate or acetate buffer.

5. A composition as claimed in Claim 4, wherein the buffer is disodium phosphate, dipotassium phosphate, sodium dihydrogen phosphate, potassium dihydrogen phosphate, phosphoric acid plus base, sodium citrate, citric acid plus base, sodium acetate or acetic acid plus base.

6. A composition as claimed in any one of Claims 2 to 5, wherein the quantity of buffer is in the range of about 1 mg/mL to about 30 mg/mL.

7. A composition as claimed in any one of the preceding claims wherein the antihistamine is selected from acrivastine, alimemazine, anatazoline, astemizole, azatadine, azelastine, bamipine, bepotastine, bromazine, bromopheniramine, buclizine, carbinoxamine, cetirizine, chlorocyclizine, chloropyramine, chlorophenamine, cinnarizine, clemastine, clemizole, clocinizine, cyclizine, cyproheptadine, deptropine, desloratadine, dexchlorpheniramine, dimenhydrinate, dimetindene, dimetotiazine, diphenhydramine, piphenylpyraline, doxylamine, ebastine, efletirizine, embramine, emedastine, epinastine, fexofenadine, fiunarizine, homochlorocyclizine, hydroxyzine, isothipendyl, levocarbastine, levocetirizine, loratadine, mebhydroline, meclozine, mepyramine, mequitazine, methdilazine, mizolastine, niaprazine, olopatadine, oxatomide, oxomemazine, pemirolast, phenindamine, pheniramine, phenyltoloxamine, pimethixene, pipinhydrinate, promethazine, propiomazine, quifenadine, rapatadine, setastine, terfenadine, thenyldiamine, thiethylperazine, thonzylamine, tolpropamine, trimethobenzamine, tripelennamine, triprolidine, tritoqualine and a pharmaceutically-acceptable salt of any of these compounds.

8. A composition as claimed in Claim 7, wherein the antihistamine is selected from loratadine, azelastine, fexofenadine, levocetirizine, cetirizine and a pharmaceutically-acceptable salt thereof.

9. A composition as claimed in Claim 8, wherein the antihistamine is cetirizine and the salt is a chloride salt, a hydrochloride salt or a nitrate salt.

10. A composition as claimed in Claim 9, wherein the salt is cetirizine dinitrate or cetirizine dihydrochloride.

11. A composition as claimed in Claim 9 or Claim 10, wherein the amount of cetirizine or salt employed in the preparation of the composition is from about 1 mg/mL to about 30 mg/mL calculated on the zwitterionic form.

12. A composition as claimed in Claim 11, wherein the amount is from about 5.5 mg/mL to about 22 mg/mL.

13. A composition as claimed in any one of the preceding claims wherein the corticosteroid is selected from alclometasone, beclometasone, betamethasone, budesonide, ciclesonide, clobetasol, clobetasone, deflazacort, deprodone, dexamethasone, diflucortolone, fluocinolone, etiprednol, flunisolide, fluocinonide, fluocortolone, fluprednidene, flurometholone, fluticasone, halcinonide, hydrocortisone, KSR 592, loteprednol, methylprednisolone, mometasone, prednisolone, rimexolone, triamcinolone and a pharmaceutically acceptable salt of any of these compounds.

14. A composition as claimed in Claim 13 wherein the corticosteroid is selected from budesonide, ciclesonide, fluticasone, triamcinolone, mometasone and a pharmaceutically acceptable salt of any of these compounds.

15. A composition as claimed in any one of the preceding claims, wherein the polar lipid is of a natural origin, is of a synthetic/semi-synthetic origin, or comprises a mixture of the two.

16. A composition as claimed in any one of the preceding claims, wherein the polar lipid comprises or consists of a phospholipid or a mixture of phospholipids.

17. A composition as claimed in Claim 16, wherein the phospholipid comprises one that is based on phosphatidylcholine, phosphatidylglycerol, phosphatidylinositol, phosphatidic acid, phosphatidylserine or a mixture thereof.

18. A composition as claimed in Claim 16 or Claim 17, wherein the phospholipid comprises one that is represented by the general formula I, 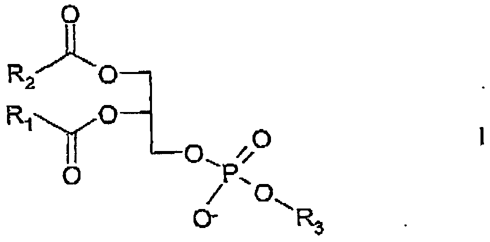 wherein R1 and R2 independently represent a saturated or unsaturated, branched or straight chain alkyl group having between 7 and 23 carbon atoms and R3 represents an amide or ester bonding group.

19. A composition as claimed in Claim 18, wherein the amide or ester bonding - group is - CH2-CH(OH)-CH2OH, -CH2-CH2-N(CHs)₃, -CH2-CH2-NH2, -H or -CH2-CH(NH2)-COOH.

20. A composition as claimed in any one of Claims 16 to 19, wherein the phospholipid comprises a membrane lipid derived from soybean.

21. A composition as claimed in Claim 20 wherein the phospholipid comprises Lipoid S75, Lipoid S100 and/or Lipoid S75-3N.

22. A composition as claimed in any one of Claims 16 to 21, wherein the phospholipid comprises dilaurylphosphatidylcholine, dipalmitoylphosphatidylcholine, dilaurylphosphatidylglycerol, dimyristolphosphatidylglycerol, dioleoylphosphatidylglycerol, dioleoylphosphatidylcholine or dimyristolphos-phatidylcholine.

23. A composition as claimed in Claim 22, wherein the phospholipid comprises dioleoylphosphatidylcholine or dimyristolphosphatidylcholine.

24. A composition as claimed in any one of Claims 1 to 15, wherein the polar lipid comprises or consists of a glycolipid or a mixture of glycolipids.

25. A composition as claimed in Claim 24, wherein the glycolipid comprises a glycoglycerolipid.

26. A composition as claimed in Claim 25, wherein the glycoglycerolipid comprises a galactoglycerolipid.

27. A composition as claimed in Claim 25, wherein the glycoglycerolipid comprises a digalactosyldiacylglycerol of the general formula II, 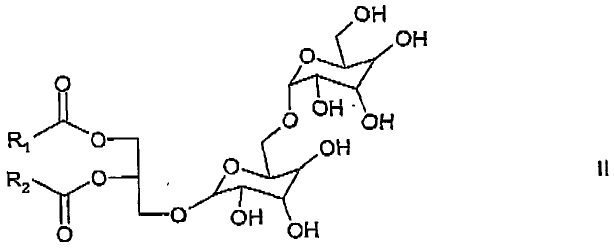 wherein R1 and R2 are as defined in Claim 18.

28. A composition as claimed in any one of Claims 24 to 27, wherein the glycolipid comprises digalactosyldiacylglycerol.

29. A composition as claimed in Claim 24, wherein the glycolipid comprises a glycosphingolipid.

30. A composition as claimed in Claim 29, wherein the glycosphingolipid comprises a monoglycosylsphingoid, an oligoglycosylsphingoid, an oligoglycosylceramide, a monoglycosylceramide, a sialoglycosphingolipid, a uronoglycosphingolipid, a sulfoglycosphingolipid, a phosphoglycosphingolipid, a phosphonoglycosphingolipid, a ceramide, a monohexosylceramide, a dihexosylceramide, a sphingomyelin, a lysosphingomyelin, a sphingosine or a mixture thereof.

31. A composition as claimed in Claim 30, wherein the glycosphingolipid comprises sphingomyelin or a product derived therefrom.

32. A composition as claimed in Claim 24, wherein the glycolipid comprises a glycophosphatidylinositol.

33. A composition as claimed in any one of the preceding claims, wherein the amount of polar lipid substance that is used is in the range of about 10 mg/mL to about 120 mg/mL.

34. A composition as claimed in any one of Claims 1 to 22 or 33, wherein the amount of phospholipid in the composition is from about 17 mg/mL to about 70 mg/mL.

35. A composition as claimed in Claim 34, wherein the amount is from about 20 mg/mL to about 40 mg/mL.

36. A composition as claimed in any one of the preceding claims, which further comprises an antioxidant, a chelating agent, a preservative or a viscosity-increasing agent.

37. A composition as claimed in Claim 36, wherein the antioxidant is alpha-tocopherol, ascorbic acid, butylated hydroxyanisole, butylated hydroxytoluene, citric acid, fumaric acid, malic acid, monothioglycerol, propionic acid, propyl gallate, sodium ascorbate, sodium bisulfite, sodium metabisulfite, potassium metabisulfite, sodium sulfite, tartaric acid and/or vitamin E; the chelating agent is ethylenediaminetetraacetic acid (and/or a salt thereof), ethylenediaminetriacetic acid and/or diethylenetriaminepentaacetic acid; the preservative is benzalkonium chloride, benzoic acid, butylated hydroxyanisole, butylparaben, chlorbutanol, ethylparaben, methylparaben, propylparaben, phenoxyethanol and/or phenylethyl alcohol; or the viscosity-increasing agent is polyethyleneglycol, crosslinked polyvinylpyrrolidone and/or hydroxypropylmethyl cellulose.

38. A composition as claimed in any one of the preceding claims, wherein the diameter of the liposomes is less than about 200 nm.

39. A composition as claimed in Claim 38, wherein the diameter is between about 40 nm and about 100 nm.

40. A process for the preparation of a composition as claimed in any one of the preceding claims, which process comprises:
(a) mixing together, in an aqueous medium, a corticosteroid, an antihistamine and a polar lipid, or a mixture of polar lipids, that is/are swellable in aqueous media; and
(b) homogenising the preparation.

41. A process as claimed in Claim 40, wherein the aqueous medium is a buffer solution.

42. A process as claimed in Claim 40 or Claim 41, wherein, prior to the homogenisation step, the pH is adjusted to the desired value by adding an acid or a base.

43. A process as claimed in any one of Claims 40 to 42, wherein, prior to the homogenisation step, water, saline or buffer solution is added to the preparation to obtain a desired final batch volume.

44. A process as claimed in Claim 43 (as dependent on Claim 42), wherein the addition of water, saline or buffer takes place after the pH adjusting step.

45. A process as claimed in any one of Claims 40 to 44, wherein at least one of the solutions/liquids is/are purged with nitrogen and/or argon.

46. A process as claimed in any one of Claims 40 to 45, wherein the lipid(s) and/or corticosteroid is/are pre-treated with organic solvent.

47. A process as claimed in any one of Claims 40 to 46, wherein the homogenisation step (b) comprises vigorous mechanical mixing, high speed homogenisation, shaking, vortexing and/or rolling.

48. A process as claimed in any one of Claims 40 to 47, which comprises an additional liposome size-reduction step.

49. A process as claimed in Claim 48, wherein the size-reduction step comprises extrusion through a membrane filter.

50. A process as claimed in any one of Claims 40 to 46, 48 or 49, wherein the homogenisation step and/or size-reduction step comprises high-pressure homogenisation.

51. A homogeneous pharmaceutical composition comprising an antihistamine, a corticosteroid, a polar lipid liposome and a pharmaceutically-acceptable aqueous carrier obtainable by a process comprising or consisting essentially of the process steps according to any one of claims 40 to 50.

52. A composition as claimed in any one of Claims 1 to 39, or 51, which is suitable for nasal, ocular and/or pulmonary delivery to a patient.

53. A composition as claimed in Claim 52, wherein the mode of delivery is nasal.

54. A composition as claimed in any one of Claims 1 to 39, or 51, for use in medicine.

55. The use of a composition as claimed in any one of Claims 1 to 39, or 51, for the manufacture of a medicament for the treatment of rhinitis, of asthma and/or of chronic obstructive pulmonary disease, which treatment comprises administration of that composition to a person suffering from or susceptible to that disorder.

56. The use as claimed in Claim 55, wherein the disorder is rhinitis.

## Patentansprüche

1. Homogene pharmazeutische Zusammensetzung, umfassend ein Antihistamin, ein Kortikosteroid, ein Liposom aus polarem Lipid und einen pharmazeutisch unbedenklichen wässrigen Träger, wobei die Wirkstoffkonzentration im wässrigen Träger, ob innerhalb oder außerhalb der liposomalen Strukturen, im Wesentlichen ähnlich ist und sich um +-10% unterscheidet, wenn man die Konzentrationen innerhalb und außerhalb der liposomalen Strukturen vergleicht.

2. Zusammensetzung nach Anspruch 1, welche weiterhin einen pharmazeutisch unbedenklichen Puffer umfasst, der dazu in der Lage ist, einen pH-Wert von etwa pH 4 bis etwa pH 8 bereitzustellen.

3. Zusammensetzung nach Anspruch 2, wobei der pH-Wert-Bereich etwa pH 5 bis etwa pH 7 beträgt.

4. Zusammensetzung nach Anspruch 2 oder 3, wobei es sich bei dem Puffer um einen Phosphat-, Citrat- oder Acetatpuffer handelt.

5. Zusammensetzung nach Anspruch 4, wobei der Puffer Dinatriumphosphat, Dikaliumphosphat, Natriumdihydrogenphosphat, Kaliumdihydrogenphosphat, Phosphorsäure plus Base, Natriumcitrat, Citronensäure plus Base, Natriumacetat oder Essigsäure plus Base ist.

6. Zusammensetzung nach einem der Ansprüche 2 bis 5, wobei die Menge an Puffer im Bereich von etwa 1 mg/ml bis etwa 30 mg/ml liegt.

7. Zusammensetzung nach einem der vorhergehenden Ansprüche, wobei das Antihistamin ausgewählt ist aus:
Acrivastin, Alimemazin, Anatazolin, Astemizol, Azatadin, Azelastin, Bamipin, Bepotastin, Bromazin, Bromopheniramin, Buclizin, Carbinoxamin, Cetirizin, Chlorocyclizin, Chloropyramin, Chlorophenamin, Cinnarizin, Clemastin, Clemizol, Clocinizin, Cyclizin, Cyproheptadin, Deptropin, Desloratadin, Dexchlorpheniramin, Dimenhydrinat, Dimetinden, Dimetotiazin, Diphenhydramin, Piphenylpyralin, Doxylamin, Ebastin, Efletirizin, Embramin, Emedastin, Epinastin, Fexofenadin, Flunarizin, Homochlorocyclizin, Hydroxyzin, Isothipendyl, Levocarbastin, Levocetirizin, Loratadin, Mebhydrolin, Meclozin, Mepyramin, Mequitazin, Methdilazin, Mizolastin, Niaprazin, Olopatadin, Oxatomid, Oxomemazin, Pemirolast, Phenindamin, Pheniramin, Phenyltoloxamin, Pimethixen, Pipinhydrinat, Promethazin, Propiomazin, Quifenadin, Rapatadin, Setastin, Terfenadin, Thenyldiamin, Thiethylperazin, Thonzylamin, Tolpropamin, Trimethobenzamin, Tripelennamin, Triprolidin, Tritoqualin und pharmazeutisch unbedenklichen Salzen dieser Verbindungen.

8. Zusammensetzung nach Anspruch 7, wobei das Antihistamin ausgewählt ist aus Loratadin, Azelastin, Fexofenadin, Levocetirizin, Cetirizin und pharmazeutisch unbedenklichen Salzen davon.

9. Zusammensetzung nach Anspruch 8, wobei es sich bei dem Antihistamin um Cetirizin handelt und das Salz ein Chloridsalz, ein Hydrochloridsalz oder ein Nitratsalz ist.

10. Zusammensetzung nach Anspruch 9, wobei das Salz Cetirizindinitrat oder Cetirizindihydrochlorid ist.

11. Zusammensetzung nach Anspruch 9 oder 10, wobei die Menge an bei der Herstellung der Zusammensetzung verwendeten Cetirizin oder Salz etwa 1 mg/ml bis etwa 30 mg/ml, berechnet für die zwitterionische Form, beträgt.

12. Zusammensetzung nach Anspruch 11, wobei die Menge etwa 5,5 mg/ml bis etwa 22 mg/ml beträgt.

13. Zusammensetzung nach einem der vorhergehenden Ansprüche, wobei das Kortikosteroid ausgewählt ist aus Alclometason, Beclometason, Betamethason, Budesonid, Ciclesonid, Clobetasol, Clobetason, Deflazacort, Deprodon, Dexamethason, Diflucortolon, Fluocinolon, Etiprednol, Flunisolid, Fluocinonid, Fluocortolon, Flupredniden, Flurometholon, Fluticason, Halcinonid, Hydrocortison, KSR 592, Loteprednol, Methylprednisolon, Mometason, Prednisolon, Rimexolon, Triamcinolon und pharmazeutisch unbedenklichen Salzen dieser Verbindung.

14. Zusammensetzung nach Anspruch 13, wobei das Kortikosteroid ausgewählt ist aus Budesonid, Ciclesonid, Fluticason, Triamcinolon, Mometason und pharmazeutisch unbedenklichen Salzen dieser Verbindung.

15. Zusammensetzung nach einem der vorhergehenden Ansprüche, wobei das polare Lipid natürlichen Ursprungs ist, synthetischen/halbsynthetischen Ursprungs ist oder eine Mischung der beiden umfasst.

16. Zusammensetzung nach einem der vorhergehenden Ansprüche, wobei das polare Lipid ein Phospholipid oder eine Mischung von Phospholipiden umfasst oder daraus besteht.

17. Zusammensetzung nach Anspruch 16, wobei das Phospholipid ein Phospholipid umfasst, das auf Phosphatidylcholin, Phosphatidylglycerin, Phosphatidylinositol, Phosphatidsäure, Phosphatidylserin oder einer Mischung davon basiert.

18. Zusammensetzung nach Anspruch 16 oder 17, wobei das Phospholipid ein Phospholipid umfasst, das durch die allgemeine Formel I wiedergegeben wird, 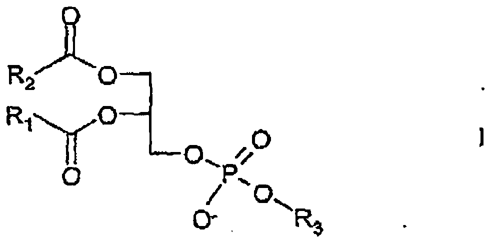 wobei R1 und R2 unabhängig voneinander für eine gesättigte oder ungesättigte verzweigte oder geradkettige Alkylgruppe mit 7 bis 23 Kohlenstoffatomen stehen und R3 für eine Amid- oder Esterbindungsgruppe steht.

19. Zusammensetzung nach Anspruch 18, wobei es sich bei der Amid- oder Esterbindungsgruppe um -CH2-CH(OH)-CH2OH, -CH2-CH2-N(CHs)3, -CH2-CH2-NH2, -H oder -CH2-CH(NH2)-COOH.

20. Zusammensetzung nach einem der Ansprüche 16 bis 19, wobei das Phospholipid ein Sojabohnen-Membranlipid umfasst.

21. Zusammensetzung nach Anspruch 20, wobei das Phospholipid Lipoid S75, Lipoid S100 und/oder Lipoid S75-3N umfasst.

22. Zusammensetzung nach einem der Ansprüche 16 bis 21, wobei das Phospholipid Dilaurylphosphatidylcholin, Dipalmitoylphosphatidylcholin, Dilaurylphosphatidylglycerin, Dimyristolphosphatidylglycerin, Dioleoylphosphatidylglycerin, Dioleoylphosphatidylcholin oder Dimyristolphosphatidylcholin umfasst.

23. Zusammensetzung nach Anspruch 22, wobei das Phospholipid Dioleoylphosphatidylcholin oder Dimyristolphosphatidylcholin umfasst.

24. Zusammensetzung nach einem der Ansprüche 1 bis 15, wobei das polare Lipid ein Glykolipid oder eine Mischung von Glykolipiden umfasst oder daraus besteht.

25. Verfahren nach Anspruch 24, wobei das Glykolipid ein Glykoglycerolipid umfasst.

26. Zusammensetzung nach Anspruch 25, wobei das Glycoglycerolipid ein Galactoglycerolipid umfasst.

27. Zusammensetzung nach Anspruch 25, wobei das Glycoglycerolipid ein Digalactosyldiacylglycerin der allgemeinen Formel II umfasst, 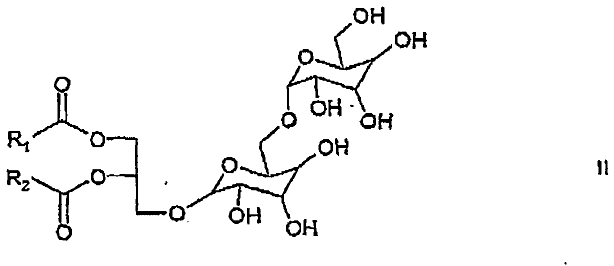 wobei R1 und R2 wie in Anspruch 18 definiert sind.

28. Zusammensetzung nach einem der Ansprüche 24 bis 27, wobei das Glykolipid Digalactosyldiacylglycerin umfasst.

29. Zusammensetzung nach Anspruch 24, wobei das Glycolipid ein Glycosphingolipid umfasst.

30. Zusammensetzung nach Anspruch 29, wobei das Glycosphingolipid ein Monoglycosylsphingoid, ein Oligoglycosylsphingoid, ein Oligoglycosylceramid, ein Monoglycosylceramid, ein Sialoglycosphingolipid, ein Uronoglycosphingolipid, ein Sulfoglycosphingolipid, ein Phosphoglycosphingolipid, ein Phosphonoglycosphingolipid, ein Ceramid, ein Monohexosylceramid, ein Dihexosylceramid, ein Sphingomyelin, ein Lysosphingomyelin, ein Sphingosin oder eine Mischung davon umfasst.

31. Zusammensetzung nach Anspruch 30, wobei das Glycosphingolipid Sphingomyelin oder ein davon abgeleitetes Produkt umfasst.

32. Zusammensetzung nach Anspruch 24, wobei das Glycolipid ein Glycophosphatidylinosit umfasst.

33. Zusammensetzung nach einem der vorhergehenden Ansprüche, wobei die Menge an verwendeter polarer Lipidsubstanz im Bereich von etwa 10 mg/ml bis etwa 120 mg/ml liegt.

34. Zusammensetzung nach einem der Ansprüche 1 bis 22 oder 33, wobei die Menge an Phospholipid in der Zusammensetzung etwa 17 mg/ml bis etwa 70 mg/ml beträgt.

35. Zusammensetzung nach Anspruch 34, wobei die Menge etwa 20 mg/ml bis etwa 40 mg/ml beträgt.

36. Zusammensetzung nach einem der vorhergehenden Ansprüche, welche weiterhin ein Antioxidationsmittel, einen Chelator, einen Konservierungsstoff oder ein die Viskosität erhöhendes Mittel umfasst.

37. Zusammensetzung nach Anspruch 36, wobei es sich bei dem Antioxidationsmittel um alpha-Tocopherol, Ascorbinsäure, Butylhydroxyanisol, Butylhydroxytoluol, Citronensäure, Fumarsäure, Äpfelsäure, Monothioglycerin, Propionsäure, Propylgalat, Natriumascorbat, Natriumhydrogensulfit, Natriumdisulfit, Kaliumdisulfit, Natriumsulfit, Weinsäure und/oder Vitamin E ist; der Chelator Ethylendiamintetraessigsäure (und/oder ein Salz davon), Ethylendiamintriessigsäure und/oder Diethylentriaminpentaessigsäure ist; das Konservierungsmittel Benzalkoniumchlorid, Benzoesäure, Butylhydroxyanisol, Butylparaben, Chlorbutanol, Ethylparaben, Methylparaben, Propylparaben, Phenoxyethanol und/oder Phenylethylalkohol ist; oder das die Viskosität erhöhende Mittel Polyethylenglykol, quervernetztes Polyvinylpyrrolidon und/oder Hydroxypropylmethylcellulose ist.

38. Zusammensetzung nach einem der vorhergehenden Ansprüche, wobei der Durchmesser der Liposomen unter etwa 200 nm liegt.

39. Zusammensetzung nach Anspruch 38, wobei der Durchmesser zwischen etwa 40 nm und etwa 100 nm liegt.

40. Verfahren zur Herstellung einer Zusammensetzung nach einem der vorhergehenden Ansprüche, wobei das Verfahren Folgendes umfasst:
(a) Vermischen eines Kortikosteroids, eines Antihistamins und eines polaren Lipids oder einer Mischung von polaren Lipiden, die in wässrigen Medien aufquellbar ist/sind, in einem wässrigen Medium und
(b) Homogenisieren der Zubereitung.

41. Verfahren nach Anspruch 40, wobei es sich bei dem wässrigen Medium um eine Pufferlösung handelt.

42. Verfahren nach Anspruch 40 oder 41, wobei vor dem Homogenisierungsschritt der pH-Wert durch Zugabe einer Säure oder einer Base auf den gewünschten Wert eingestellt wird.

43. Verfahren nach einem der Ansprüche 40 bis 42, wobei vor dem Homogenisierungsschritt der Zubereitung Wasser, Kochsalzlösung oder Pufferlösung zugesetzt wird, so dass man ein gewünschtes Chargenendvolumen erhält.

44. Verfahren nach Anspruch 43 (wie abhängig von Anspruch 42), wobei die Zugabe von Wasser, Kochsalzlösung oder Puffer nach dem Schritt, bei dem der pH-Wert eingestellt wird, stattfindet.

45. Verfahren nach einem der Ansprüche 40 bis 44, wobei mindestens eine der Lösungen/Flüssigkeiten mit Stickstoff und/oder Argon gespült wird.

46. Verfahren nach einem der Ansprüche 40 bis 45, wobei das Lipid/die Lipide und/oder das Kortikosteroid mit einem organischen Lösungsmittel vorbehandelt wird/werden.

47. Verfahren nach einem der Ansprüche 40 bis 46, wobei der Homogenisierungsschritt (b) kräftiges mechanisches Mischen, Hochgeschwindigkeitshomogenisierung, Schütteln, Vortexen und/oder Rollen umfasst.

48. Verfahren nach einem der Ansprüche 40 bis 47, welches weiterhin einen zusätzlichen, die Größe des Liposoms reduzierenden Schritt umfasst.

49. Verfahren nach Anspruch 48, wobei der größereduzierende Schritt eine Extrusion durch einen Membranfilter umfasst.

50. Verfahren nach einem der Ansprüche 40 bis 46, 48 oder 49, wobei der Homogenisierungsschritt und/oder der größereduzierende Schritt eine Hochdruckhomogenisierung umfasst.

51. Homogene pharmazeutische Zusammensetzung, umfassend ein Antihistamin, ein Kortikosteroid, ein Liposom aus polarem Lipid und einen pharmazeutisch unbedenklichen wässrigen Träger, erhältlich durch ein Verfahren, welches im Wesentlichen die Verfahrensschritte nach einem der Ansprüche 40 bis 50 umfasst oder daraus besteht.

52. Zusammensetzung nach einem der Ansprüche 1 bis 39 oder 51, welche für die nasale, okulare und/oder pulmonale Verabreichung an einen Patienten geeignet ist.

53. Verfahren nach Anspruch 52, wobei die Verabreichungsweise nasal ist.

54. Zusammensetzung nach einem der Ansprüche 1 bis 39 oder 51 zur Verwendung in der Medizin.

55. Verwendung einer Zusammensetzung nach einem der Ansprüche 1 bis 39 oder 51 zur Herstellung eines Medikaments zur Behandlung von Rhinitis, Asthma und/oder chronischer obstruktiver Atemwegserkrankung, wobei die Behandlung die Verabreichung der Zusammensetzung an eine an der Krankheit leidende Person oder eine gegenüber der Krankheit empfindliche Person umfasst.

56. Verwendung nach Anspruch 55, wobei es sich bei der Erkrankung um Rhinitis handelt.

## Revendications

1. Composition pharmaceutique homogène comprenant un antihistaminique, un corticoïde, un liposome lipidique polaire et un vecteur aqueux de qualité pharmaceutique, où la concentration en principe actif dans le vecteur aqueux est substantiellement similaire, que ce dernier soit situé à l'intérieur ou à l'extérieur des structures liposomales, et varie de plus ou moins 10 % entre les concentrations interne et externe aux structures liposomales.

2. Composition conforme à la Revendication 1, qui inclut en outre un tampon de qualité pharmaceutique pouvant mettre en place un pH compris entre environ 4 et environ 8.

3. Composition conforme à la Revendication 2, où l'intervalle de pH est compris entre environ 5 et environ 7.

4. Composition conforme à la Revendication 2 ou à la Revendication 3, où le tampon est un tampon phosphate, citrate ou acétate.

5. Composition conforme à la Revendication 4, où le tampon est le phosphate de disodium, le phosphate de dipotassium, le dihydrogénophosphate de sodium, le dihydrogénophosphate de potassium, l'acide phosphorique plus une base, le citrate de sodium, l'acide citrique plus une base, l'acétate de sodium ou l'acide acétique plus une base.

6. Composition conforme à l'une quelconque des Revendications 2 à 5, où la quantité de tampon est comprise entre environ 1 mg/mL et environ 30 mg/mL.

7. Composition conforme à l'une quelconque des revendications précédentes, où l'antihistaminique est choisi parmi les suivants : acrivastine, alimémazine, anatazoline, astémizole, azatadine, azélastine, bamipine, bépotastine, bromazine, bromophéniramine, buclizine, carbinoxamine, cétirizine, chlorocyclizine, chloropyramine, chlorophénamine, cinnarizine, clémastine, clémizole, clocinizine, cyclizine, cyproheptadine, deptropine, desloratadine, dexchlorphéniramine, dimenhydrinate, dimétindène, dimétotiazine, diphenhydramine, piphénylpyraline, doxylamine, ébastine, eflétirizine, embramine, émédastine, épinastine, fexofénadine, flunarizine, homochlorocyclizine, hydroxyzine, isothipendyle, lévocarbastine, lévocétirizine, loratadine, mebhydroline, méclozine, mépyramine, méquitazine, methdilazine, mizolastine, niaprazine, olopatadine, oxatomide, oxomémazine, pémirolast, phénindamine, phéniramine, phényltoloxamine, piméthixène, pipinhydrinate, prométhazine, propiomazine, quifénadine, rapatadine, sétastine, terfénadine, thényldiamine, thiéthylpérazine, thonzylamine, tolpropamine, triméthobenzamine, tripélennamine, triprolidine, tritoqualine ainsi que les sels de qualité pharmaceutique de n'importe lequel de ces composés.

8. Composition conforme à la Revendication 7, où l'antihistaminique est choisi parmi les suivants :
loratadine, azélastine, fexofénadine, lévocétirizine, cétirizine et leurs sels de qualité pharmaceutique.

9. Composition conforme à la Revendication 8, où l'antihistaminique est la cétirizine et le sel est un sel de chlorure, un sel de chlorhydrate ou un sel de nitrate.

10. Composition conforme à la revendication 9, où le sel est le cétirizine dinitrate ou le cétirizine dichlorhydrate.

11. Composition conforme à la Revendication 9 ou à la Revendication 10, où la quantité de cétirizine ou de sel employée dans l'élaboration de la composition est comprise entre environ 1 mg/mL et environ 30 mg/mL, calculée sur la forme zwitterionique.

12. Composition conforme à la Revendication 11, où la quantité est comprise entre environ 5,5 mg/mL et environ 22 mg/mL.

13. Composition conforme à l'une quelconque des revendications précédentes, où le corticoïde est choisi parmi les suivants : alclométasone, béclométasone, bétaméthasone, budésonide, ciclésonide, clobétasol, clobétasone, déflazacort, déprodone, dexaméthasone, diflucortolone, fluocinolone, étiprednol, flunisolide, fluocinonide, fluocortotone, fluprednidène, flurométholone, fluticasone, halcinonide, hydrocortisone, KSR 592, lotéprednol, méthylprednisolone, mométasone, prednisolone, riméxolone, triamcinolone ainsi que les sels de qualité pharmaceutique de n'importe lequel de ces composés.

14. Composition conforme à la Revendication 13, où le corticoïde est choisi parmi les suivants :
budésonide, ciclésonide, fluticasone, triamcinolone, mométasone et les sels de qualité pharmaceutique de n'importe lequel de ces composés.

15. Composition conforme à l'une quelconque des revendications précédentes, où le lipide polaire est d'origine naturelle, d'origine synthétique/semi-synthétique, ou est constitué d'un mélange des deux.

16. Composition conforme à l'une quelconque des revendications précédentes, où le lipide polaire comprend ou est constitué d'un phospholipide ou d'un mélange de phospholipides.

17. Composition conforme à la Revendication 16, où le phospholipide comprend ceux qui sont à base de phosphatidylcholine, phosphatidylglycérol, phosphatidylinositol, acide phosphatidique, phosphatidylsérine ou l'un de leur mélange.

18. Composition conforme à la Revendication 16 ou à la Revendication 17, où le phospholipide comprend l'un de ceux qui répondent à la formule générale I, 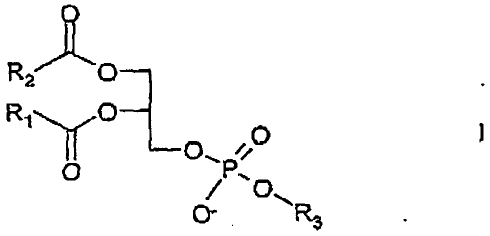 où chacun des radicaux R1 et R2 représente indépendamment un groupement alkyle à chaîne linéaire ou ramifiée, saturé ou insaturé et comportant entre 7 et 23 atomes de carbone et R3 représente un groupement liant amide ou ester.

19. Composition conforme à la Revendication 18, où le groupement liant amide ou ester est -CH2-CH(OH)-CH2OH, -CH2-CH2-N(CHs)3, -CH2-CH2-NH2, -H ou -CH2-CH(NH2)-COOH.

20. Composition conforme à l'une quelconque des Revendications 16 à 19, où le phospholipide comprend un lipide membranaire dérivé du soja.

21. Composition conforme à la Revendication 20, où le phospholipide comprend Lipoid S75, Lipoid S100 et/ou Lipoid S75-3N.

22. Composition conforme à l'une quelconque des Revendications 16 à 21, où le phospholipide comprend de la dilaurylphosphatidylcholine, de la dipalmitoylphosphatidylcholine, du dilaurylphosphatidylglycérol, du dimyristolphosphatidylglycérol, du dioléoylphosphatidylglycérol, de la dioléoylphosphatidylcholine ou de la dimyristolphosphatidylcholine.

23. Composition conforme à la Revendication 22, où le phospholipide comprend de la dioléoylphosphatidylcholine ou de la dimyristolphosphatidylcholine.

24. Composition conforme à l'une quelconque des Revendications 1 à 15, où le lipide polaire comprend ou est constitué d'un glycolipide ou d'un mélange de glycolipides.

25. Composition conforme à la Revendication 24, où le glycolipide comprend un glycoglycérolipide.

26. Composition conforme à la Revendication 25, où le glycoglycérolipide comprend un galactoglycérolipide.

27. Composition conforme à la Revendication 25, où le glycoglycérolipide comprend un digalactosyldiacylglycérol de formule générale II, 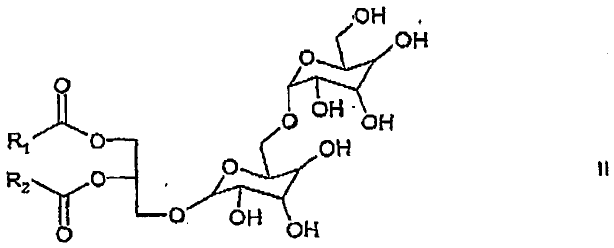 où R1 et R2 sont tels que définis dans la Revendication 18.

28. Composition conforme à l'une quelconque des Revendications 24 à 27, où le glycolipide comprend du digalactosyldiacylglycérol.

29. Composition conforme à la Revendication 24, où le glycolipide comprend un glycosphingolipide.

30. Composition conforme à la Revendication 29, où le glycosphingolipide comprend un monoglycosylsphingoïde, un oligoglycosylsphingoïde, un oligoglycosylcéramide, un monoglycosylcéramide, un sialoglycosphingolipide, un uronoglycosphingolipide, un sulfoglycosphingolipide, un phosphoglycosphingolipide, un phosphonoglycosphingolipide, un céramide, un monohexosylcéramide, un dihexosylcéramide, une sphingomyéline, une lysosphingomyéline, une sphingosine ou l'un de leurs mélanges.

31. Composition conforme à la Revendication 30, où le glycosphingolipide comprend de la sphingomyéline ou l'un de ses produits dérivés.

32. Composition conforme à la Revendication 24, où le glycolipide comprend un glycophosphatidylinositol.

33. Composition conforme à l'une quelconque des revendications précédentes, où la teneur en substance lipidique polaire employée est comprise entre environ 10 mg/mL et environ 120 mg/mL.

34. Composition conforme à l'une quelconque des Revendications 1 à 22 ou 33, où la quantité de phospholipide dans la composition est comprise entre environ 17 mg/mL et environ 70 mg/mL.

35. Composition conforme à la Revendication 34, où la quantité est comprise entre environ 20 mg/mL et environ 40 mg/mL.

36. Composition conforme à l'une quelconque des revendications précédentes, qui comprend en outre un antioxydant, un agent chélatant, un conservateur ou un agent viscosifiant.

37. Composition conforme à la Revendication 36, où l'antioxydant est l'un des suivants : alpha-tocophérol, acide ascorbique, hydroxyanisole butylé, hydroxytoluène butylé, acide citrique, acide fumarique, acide malique, monothioglycérol, acide propionique, gallate de propyle, ascorbate de sodium, bisulfite de sodium, métabisulfite de sodium, métabisulfite de potassium, sulfite de sodium, acide tartrique et/ou vitamine E ; l'agent chélatant est l'acide éthylènediaminetétracétique (et/ou l'un de ses sels), l'acide éthylènediaminetriacétique et/ou l'acide diéthylènetriaminepentacétique ; le conservateur est le chlorure de benzalkonium, l'acide benzoïque, l'hydroxyanisole butylé, le butylparabène, le chlorbutanol, l'éthylparabène, le méthylparabène, le propylparabène, le phénoxyéthanol et/ou le phényléthanol ; ou l'agent viscosifiant est le polyéthylène glycol, la polyvinylpyrrolidone réticulée et/ou l'hydroxypropylméthylcellulose.

38. Composition conforme à l'une quelconque des revendications précédentes, où le diamètre des liposomes est inférieur à environ 200 nm.

39. Composition conforme à la Revendication 38, où le diamètre est compris entre environ 40 nm et environ 100 nm.

40. Procédé d'élaboration d'une composition conforme à l'une quelconque des revendications précédentes, ledit procédé comprenant :
(a) le mélangeage dans un milieu aqueux d'un corticoïde, d'un antihistaminique et d'un lipide polaire, ou d'un mélange de lipides polaires, qui peuvent gonfler en milieu aqueux ; et
(b) l'homogénéisation de la préparation.

41. Procédé conforme à la Revendication 40, où le milieu aqueux est une solution tampon.

42. Procédé conforme à la Revendication 40 ou à la Revendication 41, où, avant l'étape d'homogénéisation, le pH est ajusté jusqu'à la valeur souhaitée par ajout d'un acide ou d'une base.

43. Procédé conforme à l'une quelconque des Revendications 40 à 42, où, avant l'étape d'homogénéisation, de l'eau, une solution saline ou une solution tampon sont ajoutées à la préparation pour obtenir un volume de lot final souhaité.

44. Procédé conforme à la Revendication 43 (en ce qu'elle dépend de la Revendication 42), où l'ajout d'eau, de solution saline ou de tampon a lieu avant l'étape d'ajustement du pH.

45. Procédé conforme à l'une quelconque des Revendications 40 à 44, où au moins l'une des solutions/l'un des liquides est purgé à l'azote et/ou à l'argon.

46. Procédé conforme à l'une quelconque des Revendications 40 à 45, où le ou les lipides et/ou le corticoïde sont prétraités par un solvant organique.

47. Procédé conforme à l'une quelconque des Revendications 40 à 46, où l'étape d'homogénéisation (b) comprend un mélangeage mécanique vigoureux, une homogénéisation à vitesse élevée, une agitation, un mélangeage au vortex et/ou un mélangeage par roulement.

48. Procédé conforme à l'une quelconque des Revendications 40 à 47, qui comprend une étape supplémentaire de réduction de la taille des liposomes.

49. Procédé conforme à la Revendication 48, où l'étape de réduction de taille comprend l'extrusion à travers un filtre à membrane.

50. Procédé conforme à l'une quelconque des Revendications 40 à 46, 48 ou 49, où l'étape d'homogénéisation et/ou l'étape de réduction de taille comprend une homogénéisation à pression élevée.

51. Composition pharmaceutique homogène comprenant un antihistaminique, un corticoïde, un liposome lipidique polaire et un vecteur aqueux de qualité pharmaceutique, pouvant être obtenue par un procédé comprenant ou consistant essentiellement en les étapes de procédé conformes à l'une quelconque des revendications 40 à 50.

52. Composition conforme à l'une quelconque des Revendications 1 à 39, ou 51, adaptée à une administration nasale, oculaire et/ou pulmonaire à un patient.

53. Composition conforme à la Revendication 52, où le mode d'administration est la voie nasale.

54. Composition conforme à l'une quelconque des revendications 1 à 39, ou 51, pour emploi en médecine.

55. Emploi d'une composition conforme à l'une quelconque des Revendications 1 à 39, ou 51, dans la fabrication d'un médicament destiné au traitement de la rhinite, de l'asthme et/ou de la bronchopneumopathie chronique obstructive, ledit traitement comprenant l'administration de cette composition à une personne souffrant de ce trouble ou y étant sensible.

56. Emploi conforme à la Revendication 55, où le trouble est la rhinite.
